# FASCICULE DE BREVET EUROPEEN

(11) **EP 0 710 639 B1**
(45) Date de publication et mention de la délivrance du brevet: **19.08.1998**
(21) Numéro de dépôt: 95402430.3
(22) Date de dépôt: 02.11.1995
(51) Int. Cl.: C07C 6/06, C07C 6/04, B01J 23/36, B01J 31/14

(54) **Nouveaux composés supportés contenant du rhénium et de l'aluminium, leur préparation et leur utilisation pour la métathèse des oléfines**
Rhenium und Aluminium enthaltende, neue, getragene Verbindungen, ihre Herstellung und Verwendung für die Metathese von Olefinen
New supported compounds containing rhenium and aluminium, their preparation and use for the metathesis of olefins

(30) Priorité: 04.11.1994 FR 9413349
(43) Date de publication de la demande: 08.05.1996
(73) Titulaire: INSTITUT FRANCAIS DU PETROLE, 92502 Rueil-Malmaison (FR)
(72) Inventeur: Commereuc, Dominique, F-92190 Meudon (FR)

(56) Documents cités:
- EP-A- 0 313 283
- EP-A- 0 444 265
- US-A- 4 454 368

## Description

La présente invention concerne de nouveaux catalyseurs supportés contenant du rhénium et de l'aluminium, leur préparation et leur utilisation pour la métathèse des oléfines.

Les catalyseurs supportés à base de rhénium actifs pour la réaction de métathèse des oléfines sont peu nombreux. Le plus ancien et le plus couramment employé est l'heptoxyde de rhénium déposé sur des oxydes minéraux, le plus souvent sur de l'alumine (brevet anglais 1 054 864), mais aussi sur des supports comportant à la fois de l'alumine et un autre cooxyde (R. Nakamura, Rec. Trav. Chim. Pays-Bas, vol. 96, 1977, p. M31). L'utilisation de cocatalyseurs organométalliques, tels que les tétraalkylétains, associés au catalyseur oxyde de rhénium sur alumine, permet, entre autres avantages, la métathèse des oléfines fonctionnelles (J.C. Mol, C. Boelhouwer *et al*., J. Chem. Soc. Chem. Comm., 1977, p. 198). W. Herrmann a revendiqué la mise en oeuvre de complexes tels que le méthyl-trioxorhénium et le pentaméthylcyclopentadiényl-trioxorhénium déposés sur des oxydes minéraux variés, dont surtout l'alumine et les silice-alumines (Hoechst, brevets européens 373 488 et 522 067).

La présente invention décrit de nouveaux catalyseurs à base de composés contenant à la fois du rhénium et de l'aluminium, déposés sur un support organique ou minéral, et qui constituent d'excellents catalyseurs pour la métathèse des oléfines.

Plus précisément, l'invention a pour objet un catalyseur supporté contenant au moins un composé du rhénium et de l'aluminium, de formule générale :

O₃Re-O-[Al(OR)(L)ₓ-O]ₙ-ReO₃

dans laquelle R est un reste hydrocarbyle contenant de 1 à 40 atomes de carbone, L est le solvant de synthèse, x est égal à 0 ou 1 et n est un nombre entier de 1 à 10.

Ces composés du rhénium et de l'aluminium répondent à la formule générale :

O₃Re-O-[Al(OR)(L)ₓ-O]ₙ-ReO₃ (A)

où R est un reste hydrocarbyle, par exemple alkyle, cycloalkyle, alkényle, aryle, et aryle ou cycloalkyle substitués, contenant de 1 à 40 atomes de carbone, de préférence un reste hydrocarbyle de 2 à 30 atomes de carbone, ce reste pouvant être substitué par au moins un groupe alkoxy ou au moins un halogène, L est le solvant de synthèse, x est égal à 0 ou 1 et n est un nombre entier de 1 à 10. A titre d'exemple, et sans que la liste soit limitative, R peut être un reste éthyle, n-propyle, isopropyle, n-butyle, t-butyle, cyclohexyle, benzyle, diphénylméthyle, phényle, méthyl-2-phényle, méthyl-4-phényle, méthoxy-2-phényle, méthoxy-4-phényle, diméthyl-2,6-phényle, diisopropyl-2,6-phényle, t-butyl-2-phényle, t-butyl-2-méthyl-4-phényle, di-t-butyl-2,6-phényle, di-t-butyl-2,6-méthyl-4-phényle, tri-t-butyl-2,4,6-phényle, phényl-2-phényle, diphényl-2,6-phényle, fluoro-2-phényle, fluoro-4-phényle, pentafluorophényle.

Le solvant L sera défini plus loin lors de la description d'un procédé de préparation.

Ces composés du rhénium et de l'aluminium sont instables, car ils sont en eux-mêmes actifs par exemple pour la catalyse de métathèse des oléfines. Pour les caractériser, il est nécessaire de les stabiliser par addition, à la fin de leur synthèse, de ligands convenables. Les composés stabilisés, inactifs en catalyse, répondent à la formule générale :

O₃Re(L')-O-[Al(OR)(L)ₓ-O]ₙ-ReO₃(L') (B)

où L' est un ligand stabilisant choisi parmi les composés comportant au moins un atome d'oxygène, de soufre, d'azote, de phosphore ou d'arsenic, par exemple un éther, comme le diéthyléther, le diméthoxy-1,2-éthane ou le tétrahydrofurane, un sulfure comme le tétrahydrothiophène, une amine, comme la triéthylamine, la pyridine, la bipyridine-2,2' ou la N,N,N',N'-tétraméthyléthylènediamine, une phosphine, comme la triphénylphosphine ou le bis-(diphénylphosphino)-1,2-éthane. R, L, x et n ont été définis lors de l'expression de la formule du composé A.

L'identification des composés B stabilisés permet de caractériser *a posteriori* les composés A : la formule de A se déduit de celle de B par abstraction du ligand stabilisant L'. Par ailleurs, par décoordination de L', le composé A peut être obtenu à partir du composé B.

Les composés de rhénium et d'aluminium utilisés dans l'invention sont synthétisés par réaction entre l'heptoxyde de rhénium Re₂O₇ et au moins un composé de l'aluminium de formule (RO)_{q}AlR'ᵣ, où R est défini comme ci-dessus, R' est un reste alkyle contenant de 1 à 20 atomes de carbone, de préférence de 1 à 6 atomes de carbone, par exemple méthyle, éthyle, isobutyle, q et r sont égaux à 1 ou 2 de telle façon que la somme q + r soit égale à 3.

La réaction est effectuée dans un solvant L, de préférence anhydre, choisi dans le groupe constitué par les hydrocarbures, aliphatiques ou aromatiques, comme par exemple le pentane, l'hexane, le benzène, le toluène, les hydrocarbures halogénés, comme par exemple le dichlorométhane, le chlorobenzène, les éthers, comme par exemple le diéthyléther, le diisopropyléther, le diméthoxy-1,2-éthane, le tétrahydrofurane ou les sulfures comme le tétrahydrothiophène. On utilise de préférence un éther. Ledit solvant L tel que défini peut entrer dans la formule générale des composés A.

Le rapport molaire entre le composé d'aluminium et le rhénium peut être choisi de 0,2:1 à 10:1. On utilise de préférence un rapport de 0,5:1 à 5:1. L'ordre d'introduction des réactifs n'est pas critique, cependant il est préférable d'introduire le composé d'aluminium dans la solution ou la suspension d'heptoxyde de rhénium.

La préparation du composé (RO)_{q}AlR'ᵣ est connue de l'homme du métier. Tout procédé de préparation de ce composé convient et par exemple, par réaction d'au moins un composé AIR'₃ avec au moins un composé ROH, R et R' étant tels que précédemment définis, la réaction ayant lieu avantageusement dans un solvant L. Le composé ainsi préparé est mis au contact de l'heptoxyde de rhénium. De façon générale, le composé ainsi préparé est isolé, puis est mis au contact de l'heptoxyde de rhénium dans les conditions décrites par l'invention.

Dans un autre mode de réalisation, les réactifs utilisés pour former le composé (RO)_{q}AlR'ᵣ sont simultanément mis au contact de l'heptoxyde de rhénium, sans qu'il y ait donc séparation du produit (RO)_{q}AlR'ᵣ.

Ainsi le catalyseur est préparé selon le procédé dans lequel on fait réagir l'heptoxyde de rhénium avec au moins un composé de formule AIR'₃ dans laquelle R' est un reste alkyle contenant de 1 à 20 atomes de carbone et avec au moins un composé de formule ROH, R étant un reste hydrocarbyle contenant de 1 à 40 atomes de carbone, la réaction ayant lieu dans un solvant L, et que le mélange obtenu est déposé sur le support, et que le produit obtenu est séché.

La température de réaction peut être de -80 à +100 °C, de préférence de -30 à +80 °C. Lorsque la réaction est terminée, on élimine avantageusement au moins en partie le solvant (par exemple on évapore le solvant sous vide) et le résidu (d'évaporation) est extrait par un solvant L₁, par exemple un hydrocarbure aliphatique, aromatique ou cycloaliphatique, ou encore un hydrocarbure halogéné ou un dérivé nitré, comme par exemple avantageusement le pentane, l'heptane, le benzène ou le toluène. La solution d'extraction est directement utilisable pour la préparation du catalyseur supporté.

Selon une variante, la solution obtenue contenant le composé A est directement utilisée pour la préparation du catalyseur supporté. Une séparation préalable du solvant est également possible dans ce cas.

Si on souhaite isoler un composé stable B qui permette de caractériser *a posteriori* le composé A présent dans la solution d'extraction, on ajoute à celle-ci le ligand L' tel que défini ci-dessus, et on sépare le composé stabilisé B par toute méthode habituelle, par exemple par précipitation ou par cristallisation.

Les composés du rhénium et de l'aluminium tels que décrits ci-dessus sont déposés sur des supports organiques ou minéraux (seuls ou en mélange), par imprégnation du support avec la solution obtenue à l'issue de leur préparation, et de préférence avec la solution d'extraction.

Les supports organiques sont en général des polymères ou des copolymères, réticulés ou non, parmi lesquels on peut citer à titre d'exemple, sans que la liste soit limitative : le polystyrène, fonctionnalisé ou non par des groupements alcool, éther, phénol, amine, phosphine, éventuellement réticulé à des taux variables par exemple par du divinylbenzène, le polychlorure de vinyle, le polyéthylène, le polybutadiène, greffés ou non par des groupements fonctionnels, les polyéthers comme les polyéthylèneglycols et leurs dérivés, les polyamides, le polyacrylonitrile, la polyvinyl-pyridine, la polyvinyl-pyrrolidinone, ainsi que des polymères naturels tels que la cellulose ou l'amidon.

Les supports minéraux sont constitués par des oxydes réfractaires et/ou des alumino-silicates qui peuvent avoir un caractère acide, neutre ou basique. On peut citer à titre d'exemple, sans que la liste soit limitative : l'alumine, la silice, les silice-alumines, les zéolithes, l'oxyde de titane, la zircone, l'oxyde de niobium, l'oxyde de chrome, la magnésie, l'oxyde d'étain.

On utilise de préférence un support minéral à caractère acide ou neutre, plus particulièrement une alumine, une silice ou une silice-alumine, ayant une surface spécifique de 10 à 400 m²/g. Le support doit au préalable être débarrassé de toute trace d'air et d'eau, par exemple par un traitement de calcination suivi d'un refroidissement sous balayage d'un gaz inerte, comme de l'azote ou de l'argon.

La méthode d'imprégnation n'est pas critique, mais il est impératif d'opérer à l'abri de l'air et de l'humidité. On peut imprégner le support par un excès de la solution, de préférence d'extraction, contenant le composé A, obtenue à l'issue de la réaction entre l'heptoxyde de rhénium et le composé de l'aluminium (RO)_{q}AlR'ᵣ. Après un temps de contact qui peut aller de quelques minutes à quelques jours, on essore le solide et on le lave au solvant pour éliminer la portion du composé qui ne s'est pas fixée. On peut aussi, dans un mode opératoire qui est préféré, utiliser la méthode d'imprégnation à sec. On ajuste alors la concentration en rhénium de la solution en fonction de la quantité de rhénium que l'on souhaite déposer sur le support, de façon que le volume de cette solution soit égal ou légèrement inférieur au volume poreux de l'échantillon de support à imprégner. L'étape d'imprégnation peut être terminée par un séchage, sous vide ou sous un courant de gaz de préférence inerte, à une température de 0 à 1000 °C, de préférence de 0 à 200 °C.

L'invention concerne donc également un procédé de préparation de catalyseurs supportés contenant au moins un composé du rhénium et de l'aluminium de formule générale :

O₃Re-O-[Al(OR)(L)ₓ-O]ₙ-ReO₃

dans laquelle R est un reste hydrocarbyle contenant de 1 à 40 atomes de carbone, L est un solvant, x est égal à 0 ou 1 et n est un nombre entier de 1 à 10, procédé dans lequel on prépare ledit composé du rhénium et de l'aluminium en faisant réagir l'heptoxyde de rhénium avec un composé de l'aluminium de formule (RO)_{q}AlR'ᵣ, dans laquelle R' est un reste alkyle contenant de 1 à 20 atomes de carbone, q et r sont égaux à 1 ou 2, et la somme q + r est égale à 3, la réaction ayant lieu dans un solvant L, à une température de -80 à +100 °C, et avec un rapport molaire entre le composé d'aluminium et le rhénium compris entre 0,2:1 et 10:1, puis ledit composé est déposé sur le support, et le produit obtenu est séché.

Le composé d'aluminium et de rhénium est donc déposé sur le support à l'aide d'une solution dudit composé dans le solvant L utilisé pour la préparation dudit composé, et/ou bien de préférence à l'aide d'une solution dudit composé dans le solvant L₁ d'extraction.

Un autre objet de la présente invention est l'utilisation de ces composés de rhénium et d'aluminium déposés sur un support comme catalyseurs pour la réaction de métathèse des oléfines. Aucune opération d'activation, chimique ou thermique, n'est nécessaire pour déclencher l'activité de ces catalyseurs. Il suffit de les mettre en contact avec une oléfine pour que la réaction de métathèse démarre.

Il est cependant possible d'ajouter au catalyseur, bien que ce ne soit pas indispensable, au moins un cocatalyseur présentant des propriétés alkylantes et/ou d'acide de Lewis. Ce cocatalyseur peut être un composé d'aluminium, de bore, de gallium, d'étain ou de plomb. On peut citer à titre d'exemple, et sans que la liste soit limitative, le trichlorure d'aluminium, le tribromure d'aluminium, le dichloroéthylaluminium, le chlorodiéthylaluminium, le triéthylaluminium, le méthylaluminoxane, l'isobutylaluminoxane, le trifluorure de bore, le trichlorure de gallium, le tribromure de gallium, le tétraméthylétain, le tétraéthylétain, le tétrabutylétain, le tétraéthylplomb. On peut aussi utiliser ces divers composés en mélange entre eux. Le cocatalyseur peut être introduit sur le catalyseur avant la métathèse, par exemple par imprégnation par l'une des méthodes décrites ci-dessus, ou pendant la métathèse, le cocatalyseur étant alors mélangé avec les oléfines.

L'invention a aussi pour objet un procédé de métathèse des oléfines en présence du catalyseur défini ci-dessus, à une température de -20 à +200 °C, de préférence de 0 à +100 °C, dans des conditions de pression variables selon que l'on souhaite conduire la réaction en phase gazeuse ou en phase liquide.

Dans une opération en phase liquide, la pression doit être suffisante pour que les réactifs et le solvant éventuel soient maintenus au moins en majorité (plus de 50 %) en phase liquide (ou en phase condensée). Le catalyseur peut alors être mis en oeuvre soit dans l'oléfine (ou les oléfines) pure, soit en présence d'un solvant constitué par un hydrocarbure aliphatique, cycloaliphatique ou aromatique, un hydrocarbure halogéné ou un dérivé nitré. On utilise de préférence un hydrocarbure ou un hydrocarbure halogéné.

Les oléfines susceptibles d'être métathétisées sont des monooléfines ayant de 2 à 30 atomes de carbone, par exemple l'éthylène, le propylène, les butènes, les pentènes, des cyclooléfines ayant de 3 à 20 atomes de carbone, par exemple le cyclopentène, le cyclooctène, le norbornène, des polyoléfines ayant de 4 à 30 atomes de carbone, par exemple l'hexadiène-1,4, l'octadiène-1,7, des cyclopolyoléfines ayant de 5 à 30 atomes de carbone, par exemple le cyclooctadiène-1,5, le norbornadiène, le dicyclopentadiène.

D'autres oléfines susceptibles d'être métathétisées sont les monooléfines ou les polyoléfines, linéaires ou cycliques, portant des groupes fonctionnels comme par exemple des halogènes ou des groupes ester. Le procédé peut également mettre en oeuvre, en cométathèse, un mélange des oléfines précédentes.

Les exemples suivants illustrent l'invention sans en limiter la portée.

### EXEMPLE 1

### Préparation du bis-(di-t-butyl-2,6-méthyl-4-phénoxy)-isobutylaluminium :

Dans un ballon de 250 ml placé sous atmosphère d'argon et muni d'un barreau magnétique, on introduit une solution de 2 ml de triisobutylaluminium dans 30 ml de pentane, puis on injecte goutte-à-goutte, sous agitation et à température ambiante, une solution de 3,49 g de di-t-butyl-2,6-méthyl-4-phénol dans 40 ml de pentane. Après environ 30 heures de réaction, le pentane est évaporé sous vide et l'analyse du solide blanc restant indique qu'il est constitué essentiellement par le bis-(di-t-butyl-2,6-méthyl-4-phénoxy)-isobutylaluminium.

### Préparation du composé de rhénium et d'aluminium (composé A) :

Dans un ballon de 250 ml placé sous atmosphère d'argon et muni d'un barreau magnétique, on introduit 2,75 g d'heptoxyde de rhénium Re₂O₇ que l'on dissout dans 40 ml de tétrahydrofurane (THF). On refroidit la solution dans un bain de carboglace-acétone et on y ajoute en 30 minutes une solution de 2,97 g de bis-(di-t-butyl-2,6-méthyl-4-phénoxy)-isobutylaluminium dans 50 ml de tétrahydrofurane. Ceci correspond à un rapport molaire Al:Re = 0,5:1. A la fin de l'addition, on laisse la température remonter à l'ambiante et on poursuit l'agitation pendant encore 2 heures. On évapore alors le solvant sous vide pour obtenir une masse solide gris-noir, que l'on extrait ensuite 5 fois par 30 ml de pentane. La solution d'extraction au pentane a une couleur brun-rouge et son évaporation à sec donne 3,32 g d'une poudre brune contenant le composé A. Cette poudre est remise en solution dans 30 ml d'heptane.

### Caractérisation du composé de rhénium et d'aluminium (composé B) :

On prélève 4,5 ml de la solution dans l'heptane du composé A préparé ci-dessus. On évapore à sec le solvant et on reprend par 10 ml de toluène. On ajoute à cette solution 0,11 g de bipyridine-2,2' (bipy) dissous dans 3 ml de toluène. Par refroidissement à -20 °C, on obtient 0,1 g du composé B sous forme de microcristaux noirs. Analyse par RMN ¹H (CD₂Cl₂) : δ [C(CH₃)₃] = 1,40 (s), δ (ρ-CH₃) = 2,23, δ (C₆H₂) = 6,95, δ (bipy coordiné) = 7,32-7,81-8,41-8,64 ppm. Analyse élémentaire : C=47,46; H=5,66; N=3,17; Al=4,33; Re=26,4 % poids, calculé pour B : O₃Re(bipy)-O-[Al(OC₆H₂-CH₃-(t-C₄H₉)₂)(THF)-O]₂-ReO₃(bipy): C=47,54; H=5,33; N=3,82; Al=3,69; Re=25,4 % poids. On en déduit que le composé A préparé ci-dessus répond à la formule : O₃Re-O-[Al(OC₆H₂-CH₃-(t-C₄H₉)₂)(THF)-O]₂-ReO₃.

### EXEMPLE 2

### Préparation du catalyseur supporté :

Le support utilisé est une alumine gamma en billes ayant une surface spécifique de 180 m²/g et un volume poreux de 0,55 ml/g. On charge 18,2 g de cette alumine dans un tube en Pyrex d'un volume utile de 100 ml placé dans un four vertical, et on la calcine à 550 °C, sous un courant d'air contenant moins de 300 ppm en poids d'eau, avec un débit de 25 I/h ramené aux conditions normales, pendant 2 heures. A la fin de cette période et avant de commencer le refroidissement, on remplace le courant d'air par un courant d'azote contenant moins de 30 ppm d'eau, et on laisse alors refroidir l'alumine dans ces conditions.

L'alumine est ensuite transférée dans un ballon de 100 ml placé sous atmosphère d'argon, et on injecte dans ce ballon, en l'agitant manuellement, 10 ml de la solution dans l'heptane du composé A préparé dans l'exemple 1. La solution est complètement absorbée par l'alumine. Après une heure, le catalyseur est séché sous vide à température ambiante. Les billes ont une coloration brun-rouge, et sont conservées sous atmosphère d'argon. La teneur en rhénium du catalyseur est de 2,2 % en poids.

### EXEMPLE 3

### Utilisation du catalyseur pour la métathèse du pentène-2 :

On prélève sous atmosphère d'argon 5,5 g du catalyseur préparé dans l'exemple 2, que l'on transfère dans un ballon de 100 ml équipé d'un barreau magnétique et plongé dans un bain thermostatique à 25 °C. On injecte alors dans le ballon 5 ml d'heptane, puis 3 ml de pentène-2 (mélange cis + trans). Après 4 minutes de réaction, la conversion du pentène-2 est de 26 %, et après 15 minutes de réaction, elle est de 50 % (ce qui représente la conversion maximum à l'équilibre thermodynamique). Les produits sont constitués uniquement par les butènes-2 cis et trans et les hexènes-3 cis et trans dans un rapport molaire butènes : hexènes = 1:1.

Le catalyseur est laissé pendant une semaine à la température ambiante au contact du mélange d'heptane, de butènes, de pentènes et d'hexènes résultant de l'essai décrit ci-dessus. Après ce laps de temps, le liquide est soutiré et le catalyseur est rincé par 3 fois 20 ml d'heptane. Le ballon contenant le catalyseur est alors de nouveau plongé dans un bain thermostatique à 25 °C. On y injecte de nouveau 5 ml d'heptane, puis 3 ml de pentène-2 (mélange cis + trans). Après 5 minutes de réaction, la conversion du pentène-2 est de 32 %, et après 15 minutes de réaction, elle est de 48 %. Les produits sont constitués uniquement par les butènes-2 cis et trans et les hexènes-3 cis et trans dans un rapport molaire butènes : hexènes = 1:1. Ce deuxième essai montre que le catalyseur ne s'est pratiquement pas désactivé au bout d'une semaine.

A l'issue de ce deuxième essai, la solution est soutirée et le catalyseur est brièvement séché sous vide puis remis sous une atmosphère d'argon, et conservé tel quel à température ambiante pendant 2 mois. Après ce laps de temps, le ballon contenant le catalyseur est de nouveau plongé dans un bain thermostatique à 25 °C. On injecte de nouveau dans le ballon 5 ml d'heptane, puis 3 ml de pentène-2 (mélange cis + trans). Après 15 minutes de réaction, la conversion est de 48 %. Les produits sont constitués uniquement par les butènes-2 cis et trans et les hexènes-3 cis et trans dans un rapport molaire butènes : hexènes = 1:1. Ce troisième essai montre que le catalyseur ne s'est pratiquement pas désactivé au bout de 2 mois.

## Revendications

1. Catalyseur supporté contenant au moins un composé du rhénium et de l'aluminium de formule générale :
O₃Re-O-[Al(OR)(L)ₓ-O]ₙ-ReO₃
dans laquelle R est un reste hydrocarbyle contenant de 1 à 40 atomes de carbone, L est le solvant de synthèse, x est égal à 0 ou 1 et n est un nombre entier de 1 à 10.

2. Catalyseur selon la revendication 1, dans laquelle R est choisi dans le groupe formé par les restes alkyle, cycloalkyle, alkényle, cycloalkyle substitué par au moins un groupe alkyle, lesdits restes étant éventuellement substitués par au moins un groupe alkoxy ou au moins un halogène.

3. Catalyseur selon la revendication 1, dans laquelle R est un reste aryle ou aryle substitué par au moins un groupe alkyle, lesdits restes étant éventuellement substitués par au moins un groupe alkoxy ou au moins un halogène.

4. Catalyseur selon l'une des revendications précédentes, dans lequel R contient de 2 à 30 atomes de carbone.

5. Catalyseur selon l'une des revendications précédentes, dans lequel R est choisi dans le groupe formé par les restes éthyle, n-propyle, isopropyle, n-butyle, t-butyle, cyclohexyle, benzyle, diphénylméthyle, phényle, méthyl-2-phényle, méthyl-4-phényle, méthoxy-2-phényle, méthoxy-4-phényle, diméthyl-2,6-phényle, diisopropyl-2,6-phényle, t-butyl-2-phényle, t-butyl-2-méthyl-4-phényle, di-t-butyl-2,6-phényle, di-t-butyl-2,6-méthyl-4-phényle, tri-t-butyl-2,4,6-phényle, phényl-2-phényle, diphényl-2,6-phényle, fluoro-2-phényle, fluoro-4-phényle, pentafluorophényle.

6. Catalyseur selon l'une des revendications précédentes, dans lequel L est choisi dans le groupe constitué par les hydrocarbures aliphatiques, les hydrocarbures aromatiques, les hydrocarbures halogénés, les éthers, les sulfures.

7. Catalyseur selon l'une des revendications précédentes, dans laquelle L est un ether.

8. Catalyseur selon l'une des revendications précédentes, dans lequel le support est choisi dans le groupe formé par les polymères, les copolymères, les polymères réticulés, les copolymères réticulés, les polymères greffés par des groupements fonctionnels, les copolymères greffés par des groupements fonctionnels, les polymères naturels, les oxydes réfractaires, les aluminosilicates, et leurs mélanges.

9. Catalyseur selon la revendication 8, dans lequel le support qui a une surface spécifique de 10 à 400 m²/g est choisi dans le groupe formé par l'alumine, la silice et la silice-alumine.

10. Procédé de préparation du catalyseur selon l'une des revendications 1 à 9, caractérisé en ce qu'on prépare le composé du rhénium et de l'aluminium en faisant réagir l'heptoxyde de rhénium avec un composé de l'aluminium de formule générale (RO)_{q}AlR'ᵣ, dans laquelle R' est un reste alkyle contenant de 1 à 20 atomes de carbone, q et r sont égaux à 1 ou 2 et la somme q + r est égale à 3, la réaction ayant lieu dans un solvant L, à une température de -80 à +100 °C, et avec un rapport molaire entre le composé d'aluminium et le rhénium compris entre 0,2:1 et 10:1, que ledit composé est déposé sur le support et que le produit obtenu est séché.

11. Procédé de préparation d'un catalyseur selon l'une des revendications 1 à 9, caractérisé en ce qu'on fait réagir l'heptoxyde de rhénium avec au moins un composé de formule AlR'₃ dans laquelle R' est un reste alkyle contenant de 1 à 20 atomes de carbone et avec au moins un composé de formule ROH, R étant un reste hydrocarbyle contenant de 1 à 40 atomes de carbone, la réaction ayant lieu dans un solvant L, et que le mélange obtenu est déposé sur le support, et que le produit obtenu est séché.

12. Procédé de préparation du catalyseur selon l'une des revendications 10 ou 11 dans lequel le solvant L est anhydre.

13. Procédé selon l'une des revendications 10 à 12, caractérisé en ce que ledit composé de rhénium et d'aluminium est déposé par imprégnation du support par une solution dudit composé.

14. Procédé selon l'une des revendications 10 à 13, caractérisé en ce que ledit composé déposé sur le support est en solution dans le solvant L de synthèse utilisé lors de la préparation dudit composé.

15. Procédé selon l'une des revendications 10 à 14, caractérisé en ce que après avoir préparé ledit composé du rhénium et de l'aluminium dans le solvant L, ledit solvant est au moins en partie éliminé et le résidu obtenu est extrait par un solvant L₁, ledit composé en solution dans le solvant L₁ est ensuite déposé sur le support.

16. Procédé selon la revendication 15, caractérisé en ce que le solvant L₁ est choisi dans le groupe constitué par les hydrocarbures aliphatiques, les hydrocarbures cycloaliphatiques, les hydrocarbures aromatiques, les hydrocarbures halogénés, les dérivés nitrés.

17. Procédé selon la revendication 15 dans lequel le solvant L₁ est choisi dans le groupe formé par le pentane, l'heptane, le benzène et le toluène.

18. Procédé de métathèse des oléfines, caractérisé en ce qu'il opère à une température de -20°C à +200°C, et en présence d'un catalyseur selon l'une des revendications 1 à 9, ou d'un catalyseur obtenu sclon l'une des revendications 10 à 17.

19. Procédé selon la revendication 18, caractérisé en ce qu'il opère entre 0 et 100 °C.

20. Procédé selon l'une des revendications 18 à 19, caractérisé en ce que les oléfines sont choisies dans le groupe formé par les monooléfines ayant de 2 à 30 atomes de carbone, les cyclooléfines ayant de 3 à 20 atomes de carbone, les polyoléfines ayant de 4 à 30 atomes de carbone, les cyclopolyoléfines ayant de 5 à 30 atomes de carbone, les monooléfines ayant de 2 à 30 atomes de carbone et portant des groupes fonctionnels choisis dans le groupe formé par les halogènes et les groupes ester, les polyoléfines ayant de 4 à 30 atomes de carbone et portant des groupes fonctionnels choisis dans le groupe formé par les halogènes et les groupes ester.

21. Procédé selon l'une des revendications 18 à 20, caractérisé en ce que les oléfines sont choisies dans le groupe formé par l'éthylène, le propylène, les butènes, les pentènes, le cyclopentène, le cyclooctène, le norbornène, l'hexadiène-1,4, l'octadiène-1,7, le cyclooctadiène-1,5, le norbornadiène, le dicyclopentadiène, l'oléate de méthyle.

22. Procédé selon l'une des revendications 18 à 21, caractérisé en ce qu'il se déroule en présence d'au moins un cocatalyseur présentant des propriétés alkylantes et/ou d'acide de Lewis.

23. Procédé selon la revendication 22, caractérisé en ce que ledit cocatalyseur est choisi dans le groupe formé par les composés d'aluminium, les composés de bore, les composés de gallium, les composés d'étain, les composés de plomb.

24. Procédé selon la revendication 22, caractérisé en ce que ledit cocatalyseur est choisi dans le groupe formé par le trichlorure d'aluminium, le tribromure d'aluminium, le dichloroéthylaluminium, le chlorodiéthylaluminium, le triéthylaluminium, le méthylaluminoxane, l'isobutylaluminoxane, le trifluorure de bore, le trichlorure de gallium, le tribromure de gallium, le tétraméthylétain, le tétraéthylétain, le tétrabutylétain, le tétraéthylplomb.

25. Procédé selon l'une des revendications 18 à 24, caractérisé en ce qu'il se déroule en phase gazeuse.

26. Procédé selon l'une des revendications 18 à 24, caractérisé en ce que les réactifs et le solvant éventuel sont au moins en partie en phase liquide.

## Patentansprüche

1. Auf einen Träger aufgebrachter Katalysator, der mindestens eine Verbindung von Rhenium und Aluminium der allgemeinen Formel enthält:
O₃Re-O-[Al(OR)(L)ₓ-O]ₙ-ReO₃
worin bedeuten:
R einen Hydrocarbyl-Rest mit 1 bis 40 Kohlenstoffatomen,
L das Synthese-Lösungsmittel,
x die Zahl 0 oder 1 und
n eine ganze Zahl von 1 bis 10.

2. Katalysator nach Anspruch 1, worin R ausgewählt wird aus einer Gruppe, die gebildet wird durch Alkyl-, Cycloalkyl-, Alkenyl-, Cycloalkyl-Reste, die durch mindestens eine Alkylgruppe substituiert sind, wobei die genannten Reste gegebenenfalls durch mindestens eine Alkoxygruppe oder mindestens ein Halogen substituiert sind.

3. Katalysator nach Anspruch 1, worin R einen Arylrest oder einen durch mindestens eine Alkylgruppe substituierten Arylrest bedeutet, wobei die genannten Reste gegebenenfalls durch mindestens eine Alkoxygruppe oder mindestens ein Halogen substituiert sind.

4. Katalysator nach einem der vorhergehenden Ansprüche, worin R 2 bis 30 Kohlenstoffatome enthält.

5. Katalysator nach einem der vorhergehenden Ansprüche, worin R ausgewählt wird aus einer Gruppe, die gebildet wird durch Ethyl-, n-Propyl-, Isopropyl-, n-Butyl-, t-Butyl-, Cyclohexyl-, Benzyl-, Diphenylmethyl-, Phenyl-, Methyl-2-phenyl-, Methyl-4-phenyl-, Methoxy-2-phenyl-, Methoxy-4-phenyl-, Dime-thyl-2,6-phenyl-, Diisopropyl-2,6-phenyl-, t-Butyl-2-phenyl-, t-Butyl-2-methyl-4-phenyl-, Di-t-butyl-2,6-phenyl-, Di-t-butyl-2,6-methyl-4-phenyl-, Tri-t-butyl2,4,6-phenyl-, Phenyl-2-phenyl-, Diphenyl-2,6-phenyl-, Fluoro-2-phenyl-, Fluoro-4-phenyl- und Pentafluorophenyl-Reste.

6. Katalysator nach einem der vorhergehenden Ansprüche, worin L ausgewählt wird aus einer Gruppe, die besteht aus aliphatischen Kohlenwasserstoffen, aromatischen Kohlenwasserstoffen, halogenierten Kohlenwasserstoffen, Ethern und Sulfiden.

7. Katalysator nach einem der vorhergehenden Ansprüche, worin L einen Ether bedeutet.

8. Katalysator nach einem der vorhergehenden Ansprüche, wobei der Träger ausgewählt wird aus einer Gruppe, die gebildet wird durch Polymere, Copolymere, vernetzte Polymere, vernetzte Copolymere, Polymere auf die funktionelle Gruppen aufgepfropft sind, Copolymere, auf die funktionelle Gruppen aufgepfropft sind, natürliche Polymere, hochschmelzende (feuerfeste) Oxide, Aluminosilicate und Mischungen davon.

9. Katalysator nach Anspruch 8, worin der Träger, der eine spezifische Oberflächengröße von 10 bis 400 m²/g hat, ausgewählt wird aus einer Gruppe, die gebildet wird durch Aluminiumoxid, Siliciumdioxid und Siliciumdioxid-Aluminiumoxid.

10. Verfahren zur Herstellung des Katalysators nach einem der Ansprüche 1 bis 9, dadurch gekennzeichnet, daß man die Verbindung von Rhenium und Aluminium herstellt, indem man Rheniumheptoxid mit einer Aluminium-Verbindung der allgemeinen Formel (RO)_{q}Al'ᵣ, worin R einen Alkylrest mit 1 bis 20 Kohlenstoffatomen bedeutet, q und r die Zahl 1 oder 2 darstellen und die Summe q + r den Wert 3 hat, bei einer Temperatur von -80 bis +100°C und mit einem Molverhältnis zwischen der Aluminium-Verbindung und dem Rhenium zwischen 0,2:1 und 10:1 reagieren läßt, wobei die Reaktion in einem Lösungsmittel L stattfindet, daß man die genannte Verbindung auf dem Träger abscheidet und das erhaltene Produkt trocknet.

11. Verfahren zur Herstellung eines Katalysators nach einem der Ansprüche 1 bis 9, dadurch gekennzeichnet, daß man Rheniumheptoxid mit mindestens einer Verbindung der Formel AlR'₃, worin R' einen Alkylrest mit 1 bis 20 Kohlenstoffatomen bedeutet, und mit mindestens einer Verbindung der Formel ROH, worin R einen Hydrocarbyl-Rest mit 1 bis 40 Kohlenstoffatomen bedeutet, reagieren läßt, wobei die Reaktion in einem Lösungsmittel L stattfindet, daß man die erhaltene Mischung auf dem Träger abscheidet und das erhaltene Produkt trocknet.

12. Verfahren zur Herstellung des Katalysators nach einem der Ansprüche 10 oder 11, bei dem das Lösungsmittel L wasserfrei ist.

13. Verfahren nach einem der Ansprüche 10 bis 12, dadurch gekennzeichnet, daß die genannte Verbindung von Rhenium und Aluminium durch Imprägnierung des Trägers mit einer Lösung der genannten Verbindung abgeschieden wird.

14. Verfahren nach einem der Ansprüche 10 bis 13, dadurch gekennzeichnet, daß die genannte Verbindung in Form einer Lösung in dem Synthese-Lösungsmittel L, das bei der Herstellung der genannten Verbindung verwendet worden ist, auf dem Träger abgeschieden wird.

15. Verfahren nach einem der Ansprüche 10 bis 14, dadurch gekennzeichnet, daß nachdem die genannte Verbindung von Rhenium und Aluminium in dem Lösungsmittel L hergestellt worden ist, das genannte Lösungsmittel mindestens zum Teil eliminiert wird und der erhaltene Rückstand mit einem Lösungsmittel L₁ extrahiert wird, wobei die genannte, in dem Lösungsmittel L₁ gelöste Verbindung anschließend auf dem Träger abgeschieden wird.

16. Verfahren nach Anspruch 15, dadurch gekennzeichnet, das das Lösungsmittel L₁ ausgewählt wird aus einer Gruppe, die besteht aus aliphatischen Kohlenwasserstoffen, cycloaliphatischen Kohlenwasserstoffen, aromatischen Kohlenwasserstoffen, halogenierten Kohlenwasserstoffen und nitrierten Derivaten davon.

17. Verfahren nach Anspruch 15, dadurch gekennzeichnet, daß das Lösungsmittel L₁ ausgewählt wird aus einer Gruppe, die gebildet wird durch Pentan, Heptan, Benzol und Toluol.

18. Verfahren zur Metathese von Olefinen, dadurch gekennzeichnet, daß man bei einer Temperatur von -20 bis +200°C und in Gegenwart eines Katalysators nach einem der Ansprüche 1 bis 9 oder eines Katalysators, wie er nach einem der Ansprüche 10 bis 17 erhalten wird, arbeitet.

19. Verfahren nach Anspruch 18, dadurch gekennzeichnet, daß es zwischen 0 und 100°C durchgeführt wird.

20. Verfahren nach einem der Ansprüche 18 bis 19, dadurch gekennzeichnet, daß die Olefine ausgewählt werden aus einer Gruppe, die gebildet wird durch Monoolefine mit 2 bis 30 Kohlenstoffatomen, Cycloolefine mit 3 bis 20 Kohlenstoffatomen, Polyolefine mit 4 bis 30 Kohlenstoffatomen, Cyclopolyolefine mit 5 bis 30 Kohlenstoffatomen, Monoolefine mit 2 bis 30 Kohlenstoffatomen, die funktionelle Gruppen tragen, ausgewählt aus einer Gruppe, die gebildet wird durch Halogene und Estergruppen, und Polyolefine mit 4 bis 30 Kohlenstoffatomen, die funktionelle Gruppen tragen, ausgewählt aus einer Gruppe, die durch Halogene und Estergruppen gebildet wird.

21. Verfahren nach einem der Ansprüche 18 bis 20, dadurch gekennzeichnet, daß die Olefine ausgewählt werden aus einer Gruppe, die gebildet wird von Ethylen, Propylen, Butenen, Pentenen, Cyclopenten, Cycloocten, Norbornen, 1,4-Hexadien, 1,7-Octadien, 1,5-Cyclooctadien, Norbornadien, Dicyclopentadien und Methyloleat.

22. Verfahren nach einem der Ansprüche 18 bis 21, dadurch gekennzeichnet, daß es in Gegenwart mindestens eines Co-Katalysators, der Alkylierungs-Eigenschaften aufweist, und/oder einer Lewis-Säure durchgeführt wird.

23. Verfahren nach Anspruch 22, dadurch gekennzeichnet, daß der genannte Co-Katalysator ausgewählt wird aus einer Gruppe, die gebildet wird von Aluminium-Verbindungen, Bor-Verbindungen, Gallium-Verbindungen, Zinn-Verbindungen und Blei-Verbindungen.

24. Verfahren nach Anspruch 22, dadurch gekennzeichnet, daß der genannte Katalysator ausgewählt wird aus einer Gruppe, die gebildet wird von Aluminiumtrichlorid, Aluminiumtribromid, Dichloroethylaluminium, Chlorodiethylaluminium, Triethylaluminium, Methylaluminoxan, Isobutylaluminoxan, Bortrifluorid, Galliumtrichlorid, Galliumtribromid, Tetramethylzinn, Tetraethylzinn, Tetrabutylzinn und Tetraethylblei.

25. Verfahren nach einem der Ansprüche 18 bis 24, dadurch gekennzeichnet, daß es in der Gasphase durchgeführt wird.

26. Verfahren nach einem der Ansprüche 18 bis 24, dadurch gekennzeichnet, daß die Reagentien und das Lösungsmittel gegebenenfalls mindestens zum Teil in flüssiger Phase vorliegen.

## Claims

1. A supported catalyst containing at least one compound of rhenium and aluminium of the general formula:
O₃Re-O- [Al(OR)(L)ₓ-O]ₙ-ReO₃
wherein R is a hydrocarbyl residue containing from 1 to 40 carbon atoms, L is the synthesis solvent, x is equal to 0 or 1 and n is an integer of from 1 to 10.

2. A catalyst according to claim 1 wherein R is selected from the group formed by the residues : alkyl, cycloalkyl, alkenyl, cycloalkyl which is substitued by at least one alkyl group, said residues being eventually substitued by at least one alkoxy groupe or at least one halogen.

3. A catalyst according to claim 1 wherein R is an aryl residue or a residue, and and aryl which is substitued by at least one alkyl group, said residues being eventually substitued by at least one alkoxy group or at least one halogen.

4. A catalyst according to one of the preceding claims wherein R contains from 2 to 30 carbon atoms.

5. A catalyst according to one of the preceding claims wherein R is selected from the group formed by the following residues: ethyl, n-propyl, isopropyl, n-butyl, t-butyl, cyclohexyl, benzyl, diphenylmethyl, phenyl, 2-methylphenyl, 4-methylphenyl, 2-methoxyphenyl, 4-methoxyphenyl, 2,6-dimethylphenyl, 2,6-diisopropylphenyl, 2-t-butylphenyl, 2-t-butyl-4methylphenyl, 2,6-di-t-butylphenyl, 2,6-di-t-butyl-4-methylphenyl, 2,4,6-tri-t-butylphenyl, 2-phenylphenyl, 2,6-diphenylphenyl, 2-fluorophenyl, 4-fluorophenyl and pentafluorophenyl.

6. A catalyst according to one of the preceding claims wherein L is selected from the group formed by aliphatic hydrocarbons, aromatic hydrocarbons, halogenated hydrocarbons, ethers and sulphides.

7. A catalyst according to one of the preceding claims wherin L is ether.

8. A catalyst according to one of the preceding claims wherein the support is selected from the group formed by polymers, copolymers, cross-linked polymers, cross-linked copolymers, polymers which are grafted by functional groups, copolymers which are grafted by functional groups, natural polymers, refractory oxides, alumino-silicates, and mixtures thereof.

9. A catalyst according to claim 8 wherein the support which has a specific surface area of from 10 to 400 m²/g is selected from the group formed by alumina, silica, and silica-alumina.

10. A process for the preparation of a catalyst according to one of claims 1 to 9 characterised in that the compound of rhenium and aluminium is prepared by reacting rhenium heptoxide with a compound of aluminium of the general formula (RO)_{q}AlR'ᵣ wherein R' is an alkyl residue containing from 1 to 20 carbon atoms, q and r are equal to 1 or 2 and the sum q+r is equal to 3, the reaction occurring in a solvent L, at a temperature of from -80 to +100°C, and with a molar ratio between the aluminium compound and the rhenium of between 0.2:1 and 10:1, said compound is deposited on the support, and the product obtained is dried.

11. A process for the preparation of a catalyst according to one of claims 1 to 9 characterised in that rhenium heptoxide is reacted with at least one compound of the formula AlR'₃ wherein R' is an alkyl residue containing from 1 to 20 carbon atoms and with at least one compound of the formula ROH, R being a hydrocarbyl residue containing from 1 to 40 carbon atoms, the reaction occurring in a solvent L, the mixture obtained is deposited on the support and the product obtained is dried.

12. A preparation process according to one of claims 10 and 11 wherein the solvent L is anhydrous.

13. A process according to one of claims 10 to 12 characterised in that said compound of rhenium and aluminium is deposited by impregnation of the support by a solution of said compound.

14. A process according to one of claims 10 to 13 characterised in that said compound deposited on the support is in solution in the synthesis solvent L used in preparation of said compound.

15. A process according to one of claims 10 to 14 characterised in that after said compound of rhenium and aluminium in the solvent L has been prepared, said solvent is at least partially eliminated and the residue obtained is extracted with a solvent L₁, and said compound in solution in the solvent L₁ is then deposited on the support.

16. A process according to claim 15 characterised in that the solvent L₁ is selected from the group formed by aliphatic hydrocarbons, cycloaliphatic hydrocarbons, aromatic hydrocarbons, halogenated hydrocarbons and nitro derivatives.

17. A process according to claim 15 characterised in that the solvent L1 is selected from the group formed by pentane, heptane, benzene, toluene.

18. A process for the metathesis of olefins characterised in that it operates at a temperature of from -20 to +200°C and in the presence of a catalyst in accordance with one of claims 1 to 9 or a catalyst obtained in accordance with one of claims 10 to 17.

19. A process according to claim 18 characterised in that it operates at between 0 and 100°C.

20. A process according to one of claims 18 and 19 characterised in that the olefins are selected from the group formed by monoolefins having from 2 to 30 carbon atoms, cycloolefins having from 3 to 20 carbon atoms, polyolefins having from 4 to 30 carbon atoms, cyclopolyolefins having from 5 to 30 carbon atoms, monoolefins having from 2 to 30 carbon atoms and bearing functional groups selected from the group formed by halogens and ester groups, and polyolefins having from 4 to 30 carbon atoms and bearing functional groups selected from the group formed by halogens and the ester groups.

21. A process according to one of claims 18 to 20 characterised in that the olefins are selected from the group formed by ethylene, propylene, butenes, pentenes, cyclopentene, cyclooctene, norbonene, hexa1,4-diene, octa-1,7-diene, cycloccta-1,5-diene, norbonadiene, dicyclopentadiene and methyl oleate.

22. A process according to one of claims 18 to 21 characterised in that it takes place in the presence of at least one co-catalyst having alkylating and/or Lewis acid properties.

23. A process according to claim 22 characterised in that said co-catalyst is selected from the group formed by aluminium compounds, boron compounds, gallium compounds, tin compounds and lead compounds.

24. A process according to claim 22 characterised in that said co-catalyst is selected from the group formed by aluminium trichloride, aluminium tribromide, dichloroethylaluminium, chlorodiethylaluminium, triethylaluminium, methylaluminoxane, isobutylaluminoxane, boron trifluoride, gallium trichloride, gallium tribromide, tetramethyltin, tetraethyltin, tetrabutyltin and tetraethyllead.

25. A process according to one of claims 18 to 24 characterised in that it takes place in a gaseous phase.

26. A process according to one of claims 18 to 24 characterised in that the reactants and any solvent are at least partially in the liquid phase.
